# EUROPEAN PATENT APPLICATION

(11) **EP 1 941 869 A1**
(43) Date of publication of application: **09.07.2008**
(21) Application number: 07100095.4
(22) Date of filing: 04.01.2007
(51) Int. Cl.: A61K 9/08, A61J 1/00, A61K 33/26

(54) **Multicompartment bag for storage of iron preparations**

(71) Applicant: VIFOR (INTERNATIONAL) AG, 9001 St Gallen (CH)
(72) Inventor: Weibel-Furer, Ludwig, 9104 Waldstatt (CH)
(74) Representative: Gille Hrabal Struck Neidlein Prop Roos

(57) **Abstract**

The present invention relates to multicompartment bags, for storage, in particular long-term storage, of iron preparations, in particular high-dose iron preparations, which are intended for parenteral administration to a patient in need of such iron preparations.

## Description

### Field of the Invention

The present invention relates to multicompartment bags, for storage, in particular long-term storage, of iron preparations, in particular high-dose iron preparations, which are intended for parenteral administration to a patient in need of such iron preparations.

### Background

Adequate supply of iron is critical to the survival and well-being of humans and many animals. Iron deficiency anemia may arise under a variety of conditions such as during pregnancy but also due to illnesses such as for example end stage renal disease (ESRD). Furthermore, in chronic diseases such as rheumatoid arthritis, certain haemolytic diseases and cancer, there is a maldistribution of body iron leading to a state of chronic anemia.

Yet, methods in the prior art for delivering iron preparations to a patient have proven largely impractical and unsatisfactory

For example, oral iron administration typically includes administration of oral iron supplements in relatively large doses, usually as various organic and inorganic salts, for example, ferrous gluconate, ferrous citrate, ferrous sulfate, ferrous fumarate and ferric-polysaccharide complexes. Oral iron administration, however, has several disadvantages, including patient noncompliance, gastrointestinal side effects, interactions with other oral medications and very poor absorption, in particular in ESRD patients, which markedly limit its effectiveness.

Intravenous administration includes for example iron-dextran preparations, which has shown to be non-toxic , but may cause severe allergic reactions, fever and rashes during injection and therefore requires slow administration with an initial test dose (e.g., INFED®; CosmoFer®;; see also for example US 5,624,668). Moreover, dextran is thought to be immunogenic, even to patients that have not been treated with dextran previously, because of cross-reactivity of dextran to native antibodies formed to polysaccharides produced by intestinal organisms. Additionally, only about half of the iron in iron dextran is bio-available after intravenous injection for red cell production. The fate of the rest is unknown. Other iron complexes for intravenous administration include for example ferric gluconate (Ferrlecit®) which may also cause severe side effects, may only be administered at low doses and needs slow administration. Moreover, each of these intravenous iron preparations is typically very expensive, in particular due to the necessity for extensive sterilization of the injectant, and their administration is time-consuming and labour-intensive.

Intramuscular administration includes for example iron dextrins and iron dextrans (e.g. Jectofer®). However, as a result of their high molecular weights, absorption in the human or animal body is unpredictable and incomplete. Furthermore, the administration of these compositions intramuscularly is clearly dose-limiting, painful and often results in local complications including an undesirable discoloration at the injection site.

Further iron preparations for e.g. parenteral administration are for example disclosed in US 3,686,397, US 6,977,249, US 3,886,267, and US 5,177,068.

In view of the above, there is a lack in the art of suitable methods for iron delivery, in particular iron preparations for infusion at optimal dosage ranges. Iron preparations for infusion purposes have shown to be unstable at low concentrations, yet in high concentrations they are incompatible with an administration by infusion. Thus, there is clearly a great need in the art for providing suitable methods for iron delivery and in particular iron preparations for infusion at optimal concentration ranges, which in specific allow administration of higher doses in a shorter amount of time.

The present invention addresses those problems by providing multicompartment bags for iron delivery, in particular high-dose iron preparations for administration by infusion.

Multicompartment bags for medical purposes are known in the art, typically for storage and administration by infusion of parenteral solutions such as infusible nutrition mixtures, amino acid solutions and the like (EP 0 875 231, US 6,398,771, EP 0 893 982, EP 0 774 348). Yet administration of highly complex and sensitive preparations such as iron preparations by infusion remained cumbersome and time-consuming. It has now been found that the multicompartment bags of the invention can be used for storage of highly complex and sensitive preparations such as iron preparations, which are known for their instability in low concentrations, e.g. as solutions for infusion. Careful consideration had to be given to the nature of the iron preparations, such as in particular optimal concentration range. Other factors may include the nature of the materials, such as compatibility, barrier capability (e.g. against oxygen), processability, and the like, to achieve sufficient long-term stability of the final product, ease of administration for a user, high degree of safety for the patient as well as satisfactory patient compliance.

In particular it has been found that the multicompartment bag of the present invention allowed storage of iron preparations at appropriate concentrations in compartments independent of their volume sizes, thus both small as well as large volumes were compatible.

### Summary of the Invention

The present invention relates to multicompartment bags, for storage, in particular long-term storage, of iron preparations, in particular high-dose iron preparations, which are intended for parenteral administration to a patient in need of such iron preparations, in particular for administration by infusion.

In particular, the present invention provides in a first aspect a multicompartment bag comprising two sheets of a polymer material that are welded peripherally and in an intercompartmental region to form at least two compartments separated by a breakable weld, characterized in that one of the at least two compartments comprises a stabilized Fe(III) preparation.

More specifically the present invention provides a multicompartment bag for long term storage of a stabilized Fe (III) preparation and suitable diluents for parenteral administration in separate compartments, which otherwise in their final mixed parenterally administrable form would be rapidly perishable. Subsequent parenteral administration is easily achieved by simply breaking the separating weld(s) and homogenously mixing the contents of the separate compartments to obtain the administrable form.

In a specific embodiment said stabilized Fe (III) preparations represent a stabilized Fe(III)-solution comprising Fe (III) ions in admixture or complexed with a ligand, preferably a carbohydrate and derivatives thereof. Said stabilized Fe(III) preparations are soluble in an aqueous medium, sufficiently stable in specific concentrations, and yet can be well absorbed by blood and the living body upon dilution with a suitable diluent and administration according to well-known techniques for parenteral administration, in particular for administration by infusion.

In a specific embodiment the multicompartment bag comprises at least one compartment with a volume ranging from 10 to 100 ml. In another embodiment the bag comprises in addition at least one compartment with a volume ranging from 50 to 1000 ml.

In a further embodiment one or more compartments of the multicompartment bag comprise separately one or more aqueous diluents suitable for infusion selected from carbohydrates, lipids, electrolytes, vitamins and mixtures thereof.

In a further embodiment the concentration of the stabilized Fe(III)-preparation within the compartment of the multicompartment bag ranges from 2 to 10 % by weight.

In yet a further embodiment the multicompartment bag has one or more inlet and outlet ports, in a preferred embodiment the multicompartment bag has one port only for use as exit port system for parenteral administration, in particular for administration by infusion.

In another aspect the present invention provides a method for delivery of a high dose iron preparation to an iron-deficient patient using a multicompartment bag according to the present invention.

In yet another aspect the present invention relates to a method of preparing a multicompartment bag having at least two compartments comprising in a first step filling the stabilized Fe(III) preparation with or without an inlet port into one of the at least two compartments, and optionally sealing the compartment thereby removing said inlet port, and in a second step filling one or more aqueous diluents suitable for infusion separately into one or more compartments of said multicompartment bag.

In a further aspect, the multicompartment bag may be enclosed in a substantially oxygen impermeable outer sealing envelope optionally in combination with a suitable oxygen-absorber.

Further objects, features, and advantages of the present invention shall become apparent from the detailed figures and description provided hereinafter.

### Figures

- Figure 1: schematic plan view of a multicompartment bag having two compartments and one fill and outlet port, enclosed in an overwrap, with an oxygen absorber placed between the bag and the overwrap, prior to mixing of the contents of the two compartments.
- Figure 2: schematic plan view of a multicompartment after mixing of the contents of the two compartments and ready for administration by infusion through the outlet port

### Detailed Description of the Invention

The present invention overcomes problems in the prior art associated with delivering iron to a patient. Iron delivery according to the present invention is accomplished by providing suitable multicompartment bags, for delivering stabilized Fe(III) preparations to an iron-deficient patient via infusion techniques.

In particular, the present invention provides in a first aspect a multicompartment bag comprising two sheets of a polymer material that are welded peripherally and in an intercompartmental region to form at least two compartments separated by a breakable weld, characterized in that one of the at least two compartments comprises a stabilized Fe(III) preparation.

A "stabilized Fe (III) preparation" as used herein, is intended to designate a composition comprising one or more Fe (III) ions in admixture with or complexed with a ligand, preferably a carbohydrate (and derivatives thereof).

As used herein, the term "a carbohydrate (and derivatives thereof)" includes, but is not limited to, monosaccharides, disaccharides, trisaccharides, oligosaccharides and their corresponding sugar alcohols, polyhydroxy compounds such as carbohydrate derivatives and chemically modified carbohydrates, hydroxyethyl starch and sugar copolymers. Both natural and synthetic carbohydrates are suitable for use herein. Synthetic carbohydrates include, but are not limited to, those which have the glycosidic bond replaced by a thiol or carbon bond. Both D and L forms of the carbohydrates may be used. The carbohydrate may be non-reducing or reducing.

Preferred carbohydrates according to the present invention include sucrose, gluconic acid, dextran, mannitol, dextrin, agarose, cellulose, maltodextrin and derivatives thereof, preferably maltodextrin.

Thus, preferred "stabilized Fe (III) preparations" include iron(III)sucrose, iron(III)gluconate, iron(III)dextran, iron(III)maltodextrin, Ferumoxytol (Polyglucose sorbitol carboxymethyl ether-coated non-stoichiometric magnetite)and the like, most preferred are iron(III)maltodextrin preparations, such as for example thosedescribed e.g. in WO 2004/037865 (whose content is fully incorporated herein), which are in form of a water-soluble iron-carbohydrate complex obtained from an aqueous iron(III)-salt solution and an aqueous solution of the product obtained by oxidizing one or several maltodextrins with an aqueous hypochlorite solution at an alkaline pH value. In a preferred embodiment stabilized Fe(III)-preparations have a concentration ranging from 2 to 10 % by weight, more preferably 2.5 to 5 %.

A "multicompartment bag" as used herein, is intended to designate a bag comprising at least two compartments, preferably two to four compartments, more preferably two or three compartments. In one embodiment one of the compartments has a small volume, e.g. a volume of below 200 ml, preferably ranging from 10 to 100 ml, more preferably 20 to 50 ml. In another embodiment one of the compartments has a volume of below 2000 ml, preferably ranging from 10 to 1000ml, more preferably 50 to 150ml. In a specific embodiment the multicompartment comprises at least one compartment with a volume ranging from 10 to 100 ml and at least one compartment with a volume ranging from 50 to 1000 ml. Preferably the volumes are chosen such, that a total reconstituted volume of 100 ml or less is obtained. Specific examples are shown in the Examples section. In a specific embodiment the multicompartment bag of the present invention comprises in addition in one or more further separate compartment one or more aqueous diluents suitable for infusion. Preferably the multicompartment bag of the present invention comprises in addition in one further separate compartment one aqueous diluent suitable for infusion.

A "diluent suitable for infusion" as used herein, is intended to designate preferably any aqueous diluent suitable for dilution of a stabilized Fe (III) preparation of the invention and may be selected from carbohydrates, lipids, electrolytes (e.g. physiological saline), vitamins and mixtures thereof, preferably physiological saline.

A "polymer material" as used herein, is intended to include a material comprising one or more polymers, which have to fulfil the requirements necessary to be processed into a bag for medical purposes, such as parenteral administration, in particular administration by infusion. First the polymer material or more specifically the inner surface of the material facing the preparations stored in the bag, must be compatible with the stored preparations, which includes that the polymer material must not be either reactive with the stored preparations or cause physical changes to the stored preparations such as coagulation, precipitation and the like, leading to decomposition and loss of activity of the stored preparations, problems which are typically associated with iron preparations. Second, the polymer material must be mechanically stable under the conditions of its intended use, e.g. it should be able to permanently weld to form a bag as well as form breakable welds, in case of the intercompartmental divisions. Third, the polymer material must be thermally stable under the conditions of its intended use, e.g. it should be sterilizable (by steam, radiation or any other known method of the art). Yet, the material should be substantially impermeable for water vapor during steam sterilization, but need not be airtight according to the present invention, when an outer sealing envelope is used in combination with an oxygen absorber. In fact if the material does permit an oxygen transfer, all residual oxygen dissolved in the stored products would be absorbed by the oxygen absorber. Likewise, the material in form of a bag must maintain its flexibility and not become fragile or brittle in particular after sterilization and/or storage.

It is understood, that if the polymer material comprises one polymer only, said polymer has to fulfil all three above requirements. If the polymer comprises more than one polymer in form of a multilayer (see hereinafter), then the polymer material representing the innermost layer must only fulfil the first requirement of inertness, whereas at least one, preferably all polymer materialls should fulfil each or both of the second and third requirement of mechanical and thermal stability.

In addition, the material may be transparent, opaque or colored. Preferably, the material is transparent and does not tend to be discolored or opaque after sterilization. Lastly, the polymer material should also be environment friendly and preferably easily recyclable.

A polymer material for use as a bag according to the present invention is preferably in form of a film with suitable characteristics such that both permanent welds and different peelable seal welds can be formed when subjected to different welding conditions or operations.

Such a polymer material according to the present invention which is chemically inert to the stored agents, autoclavable, weldable, sufficiently flexible and possible to recycle, may be formed from one or more polymers and in form of one single layer or a multilayer film. If more than one polymer is used they may be in form of separate polymer layers forming a multilayer film, or they may be compounded to form one single layer.

Thus in one embodiment the film is in form of a single layer and is made using a single polymer or a polymer mixture, which may be selected from polyethylene of high density, polypropylene, blocked polyether/polyamide or mixtures of polyethylene and styrene/ethylene/butyl, polyethylene and ethylene vinyl acetate, linear polyethylene, polypropylene and styrene/ethylene/butyl. Such materials are used to preferably make a single sheet (or alternatively the inner layer of a composite sheet).

In another embodiment such a film is a multilayer film and may be made of at least two different polymer layers of which the inner or innermost sealant layer is based on polymers that are chemically inert to the stored agents, autoclavable, weldable and possible to recycle, and the outer or outermost sealant layer provides sufficient flexibility. Typical examples of polymers to be used as the inner or innermost layer include for example polyolefins, such as polyethylenes or polypropylenes of various qualities. The terms "polyethylenes" and "polypropylenes" are intended to include both homopolymers and copolymers having such mentioned characteristics unless otherwise is specified. Typical polyethylenes may include linear low density polyethylene (LLDPE), low density polyethylene (LDPE), medium density polyethylene (MDPE), ethylene vinyl alcohol (EVOH)-copolymer, polyethylene terephthalate (PET)-copolymer, ethylene vinyl acetate copolymer (EVA), ethylene-methyl acrylate copolymer (EMA), or high density polyethylene (HDPE). Typical examples of polymers to be used as the outer or outermost layer may be any known elastomer and includes for example polyamides, polyesters and copolymers thereof (copolyesters) and in particular cycloaliphatic polyesters.

In a preferred embodiment the bag comprises films or sheets based on multiple layers, e.g. at least two layers, i.e. an inner and an outer layer, preferably three layers, i.e. further including a middle layer. Typically, the at least two layers are bonded by tie layers. The materials of all layers, especially inner and outer layers, may typically be copolymers, modified polymers and within one layer a mixture of again different types of the same polymer. These polymers include blends of one or more ethylenic homopolymer or copolymers and a propylene homopolymer or copolymer, preferably selected from polypropylene copolymer, ethylene vinyl alcohol (EVOH)-copolymer, and polyethylene terephthalate (PET)-copolymer. Such films or sheets may be bonded by extrusion lamination as described in the art. Specific Examples are shown in the Examples section

The production of multicompartment bags comprising two or more compartments separated by breakable welds according to the invention as well as the filling thereof may be done according to well-known methods known in the state of the art, especially according to for example EP 0 353 193, as well as EP 0 875 231, US 6,398,771, EP 0 893 982, EP 0 774 348, US 5,128,414 , EP 0 444 900, which documents hereby are incorporated as references.

A particularly preferred multicompartment bag will be discussed in greater detail below.

As mentioned hereinabove, the multicompartment bag may be enclosed in a substantially oxygen impermeable outer sealing envelope. Such an envelope will provide sterility, mechanical protection as well as act as an additional barrier against steam, for example if final sterilization involves steam sterilization. In the space between the multicompartment bag and the envelope, it is possible to place an oxygen scavenging composition to consume residual oxygen and the small amounts of oxygen penetrating through the envelope. The oxygen scavenging composition can be present e.g. as a separate sachet. Alternatively the oxygen scavenging composition may also be compounded as a part of a multilayered film, see for example US 5,660,761 and 6,544,611. This will reduce the risk of decomposition of the materials, in particular if long-term storage is desired. Furthermore, an oxygen indicator may be placed between the envelope and the multicompartment bag through which the transparent envelope visually indicates an oxygen leakage, for example by a color change (US 6,627,443). A suitable and effective oxygen absorber shall in addition be capable to withstand a sterilization procedure as used in the present invention, such as sterilization by steam or irradiation, without being impaired. It will be obvious to a skilled person to select a suitable oxygen absorber in an appropriate amount for a bag according to the present invention. Preferably, the oxygen absorber according to the present invention is iron containing and dependent on water for its oxygen consumption. The oxygen absorber can either be present in the container as a sachet or it can be compounded as a part of a multilayered film. It is preferred to use an oxygen absorber having a ferrous oxygen scavenging composition enclosed in one or several sachets or tray-like carriers placed between the bag and the surrounding airtight envelope in a controlled atmosphere. The skilled person will have no difficulties in obtaining suitable oxygen absorbers in an appropriate amount when designing a container according to the present invention. An estimation of a necessary quality and amount can easily be performed from its oxygen predetermined consuming capacity when given values of the container, for example, of the volume of the stored material and the oxygen barrier capacity of the surrounding envelope. For example, if the total capacity of the oxygen absorber is at least 100 ml pure oxygen, this value must be higher than the amount expected to penetrate the envelope through a given area during a given time if the envelope is made of a material having an oxygen permeability of not exceeding 5 ml oxygen per m², atm and day at a normal relative humidity. Suitable oxygen absorbers, according to the present invention, are for example described in US 4,127,503. A preferred example thereof are e.g. the Ageless™ oxygen scavengers, which contain fine iron powder covered with sea salt and a natural zeolith impregnated with a NaCl-solution (available from Mitsubishi). Other suitable oxygen scavengers include scavengers based on a powerful inorganic desiccant such as the RP System™- K Type containing unsaturated organic compounds and some inorganic sorbents like graphite and Ca(OH)₂ as well as polyethene, or the RP System™- A Type containing similar materials but also zeolith as a very strong sorbent for humidity (both available from Mitsubishi.

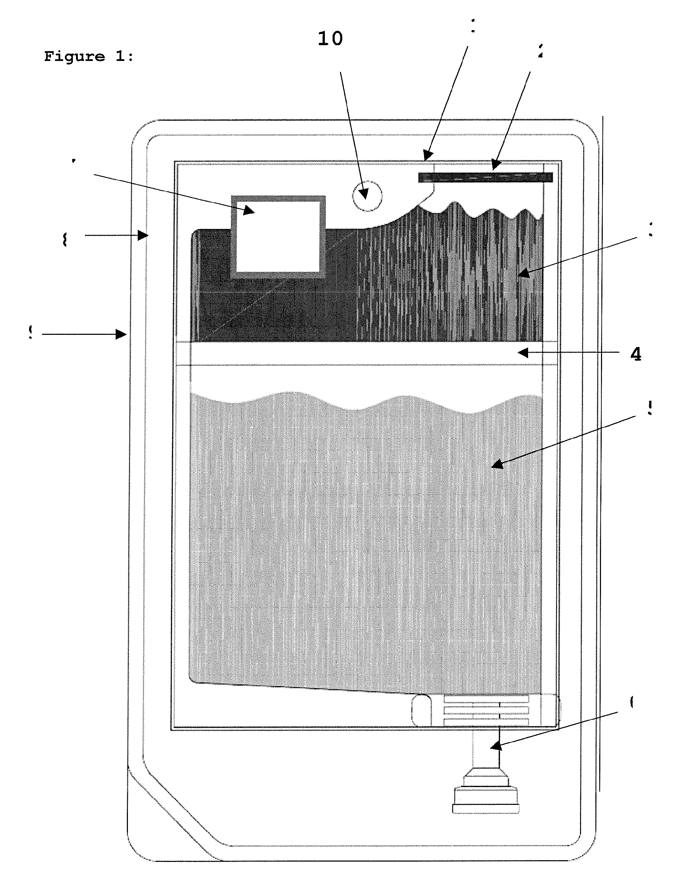

One specific embodiment of a multicompartment bag of the invention is illustrated in Figures 1 (prior to use) and 2 (ready for administration). Figure 1 shows a multicompartment bag 1 (indicated by the dotted lines) comprising an upper compartment 3 and a lower compartment 5 separated by a breakable weld 4. Filling of the container may be accomplished using several techniques. In an exemplary process, filling of the upper compartment 3 having no port includes that during the sealing process a portion of the periphery of the upper compartment 3 is left unsealed. Subsequently a vacuum is applied through suitable suction cups such that the unsealed portion and the two sheets of the polymer bag making up the upper compartment remain in an open position ready for filling. The upper compartment 3 is then filled with an iron solution through the unsealed portion using a filling needle, the unsealed portion of the upper compartment 3 is sealed by a weld 2 and the vacuum applied through the suction cups is removed. The additional diluent, i.e. a solvent such as physiological saline, is filled into the lower compartment 5 through a fill port 6, which ultimately serves as the outlet port for the infusion solution obtained by mixing the contents of the two compartments 3 and 5. The port may be closed by conventionally used stoppers. The container is then subjected to sterilization. The multicompartment 3 is enveloped in an outer bag 9 (or overwrap), which is sealed by a peel 8 to enhance the barrier and to secure sterility. Optionally an oxygen adsorber 7 may be placed between the overwarp 9 and the multicompartment bag 1, for example in form of a sachet. In addition the multicompartment bag 1 has optionally a handle part 10 (i.e. a hole) at its top to facilitate conventional administration from a hanging position.

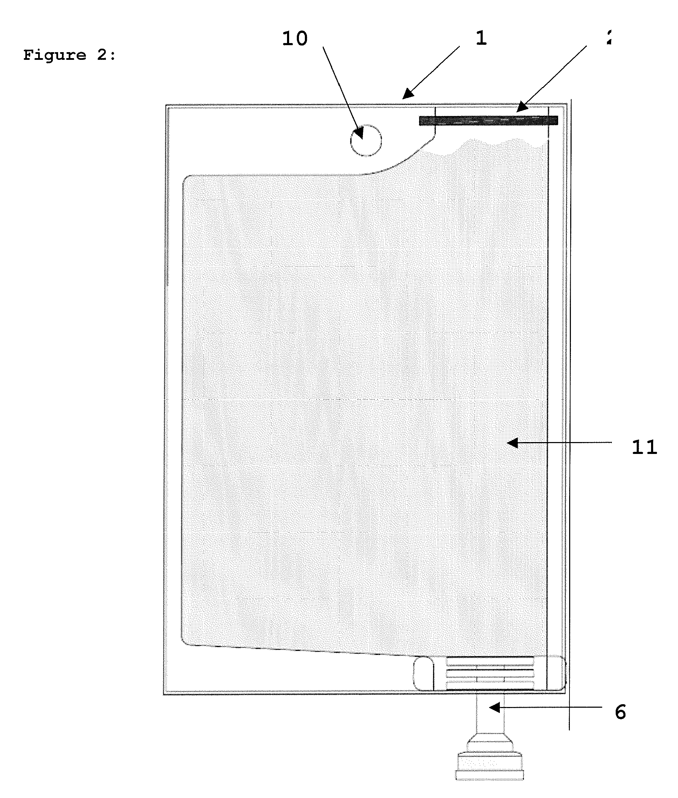

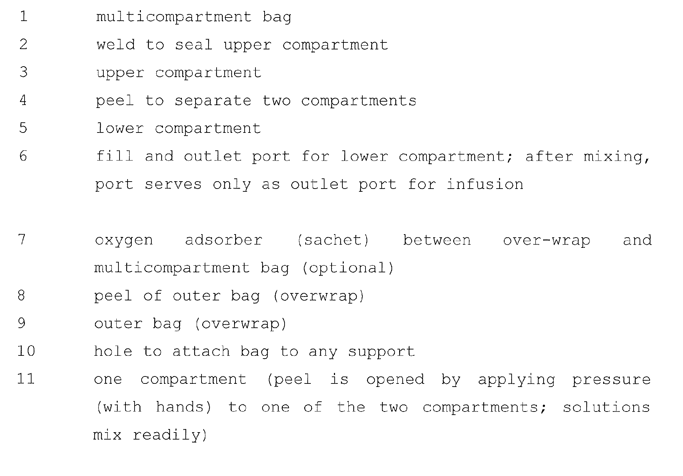

Prior to use of the infusion solution, the outer bag 9 can be removed by opening the peel 8. As shown schematically in Figure 2, the breakable weld 4 separating the two compartments 3 and 5 can be ruptured by a user applying pressure (e.g. manually) to one of the two compartments 3 and 5 to give one single mixing compartment 11, wherein the solutions can be mixed readily to give the desired infusion solution, which is subsequently ready for administration by infusion using port 4.

Preferably the Fe(III) preparation is filled into the upper compartment and a suitable diluent, preferably selected from carbohydrates, lipids, electrolytes, vitamins and mixtures thereof, is filled into the lower compartment.

The invention is further described in the following specific Examples. It is understood that these Examples are illustrative and no limitation of the scope of the invention is thereby intended. It is understood that any substitutions and/or modifications in the described invention, and any further applications of the principles of the invention as described herein which do not depart from the gist of the invention are contemplated herein as well.

### Examples

### Example 1:

Selected examples for volumes and concentrations for the bags according to the invention are shown in Table 1:

**Table 1:**

| **Volume of physiological** | **Volume of iron solution** | **Concentration of iron solution** |
|---|---|---|
| 80 ml | 10 ml | 2 % m/v |
| 250 ml | 10 ml | 4 % m/v |
| 80 ml | 20 ml | 2.5 % m/v |
| 80 ml | 20 ml | 5 % m/v |
| 80 ml | 20 ml | 10 % m/v |
| 100 ml | 20 ml | 5 % m/v |
| 250 ml | 20 ml | 5 % m/v |
| 50 ml | 50 ml | 2 % m/v |
| 50 ml | 50 ml | 4 % m/v |
| 200 ml | 50 ml | 4 % m/v |

### Example 2:

Specific examples for films comprising three layers (an outer, a middle and an inner layer) bonded by tie layers are shown in the following Table 2:

**Table 2:**

| **Function** | **Example 1** | **Example 2** | **Example 3** | **Example 4** |
|---|---|---|---|---|
| outer layer | PET | PP | PP | PET |
| tie layer | PP modified | PP modified | PP modified | PP modified |
| middle layer | EVOH | EVOH | COC | EVOH |
| tie layer | PP modified | PP modified | PP modified | PP modified |
| inner layer | PP | PP | PP | HDPE |

As indicated hereinabove, in a film according to table 2 the inner layer must be chemically inert and one or all three layers should fulfil the mechanical and thermal requirements.

## Claims

1. Multicompartment bag comprising two sheets of a polymer material that are welded peripherally and in an intercompartmental region to form at least two compartments separated by a breakable weld, **characterized in that** one of the at least two compartments comprises a stabilized Fe(III) preparation.

2. Multicompartment bag according to claim 1, **characterized in that** the stabilized Fe(III) preparation is a stabilized Fe(III)-solution in form of an admixture with or a complex with a ligand.

3. Multicompartment bag according to claims 1 or 2, **characterized in that** the ligand is a carbohydrate.

4. Multicompartment bag according to claims 2 or 3, **characterized in that** the ligand is selected from sucrose, gluconate, dextran, mannitol, dextrin, agarose, cellulose, maltodextrin and derivatives thereof.

5. Multicompartment bag according to anyone of claims 2 or 3, **characterized in that** the ligand is maltodextrin.

6. Multicompartment bag according to claims 1 to 4, **characterized in that** the stabilized Fe(III)-preparation is a water-soluble iron-carbohydrate complex obtained from an aqueous iron (III)-salt solution and an aqueous solution of the product obtained by oxidizing one or several maltodextrins with an aqueous hypochlorite solution at an alkaline pH value.

7. Multicompartment bag according to any preceding claim, **characterized in that** one or more of the at least two compartments comprise separately one or more aqueous diluents suitable for infusion selected from carbohydrates, lipids, electrolytes, vitamins and mixtures thereof.

8. Multicompartment bag according to any preceding claim comprising two compartments for separately containing a stabilized Fe(III)-solution and an aqueous diluent suitable for infusion selected from carbohydrates, lipids, electrolytes, vitamins and mixtures thereof.

9. Multicompartment bag according to any preceding claim, **characterized in that** the volume of at least one compartment ranges from 10 to 100 ml.

10. Multicompartment bag according to claim 8, wherein the at least one compartment comprises a stabilized Fe(III)-solution.

11. Multicompartment bag according to any preceding claim, wherein at least one compartment has a volume ranging from 10 to 100 ml and at least one compartment has a volume ranging from 50 to 1000 ml.

12. Multicompartment bag according to any preceding claim having a first compartment with a volume ranging from 10 to 100 ml and a second compartment with a volume ranging from 50 to 1000 ml, wherein the first compartment comprises a stabilized Fe(III)-solution and the second compartment comprises an aqueous diluent suitable for infusion.

13. Multicompartment bag according any preceding claim, **characterized in that** the concentration of the stabilized Fe(III)-preparation ranges from 2 to 10 % by weight.

14. Multicompartment bag according to any preceding claim, **characterized in that** the polymer material is in form of a single layer or a multilayer

15. Multicompartment bag according to any preceding claim, **characterized in that** the polymer material is selected from one or more polymers rendering the polymer material substantially impermeable to oxygen.

16. Multicompartment bag according to claim 14, **characterized in that** the one or more polymers are selected from ethylenic homo- or copolymers and propylene homo- or copolymers.

17. Multicompartment bag according to claims 14 or 15, **characterized in that** the one or more polymers represent polypropylene copolymer, ethylene vinyl alcohol (EVOH)-copolymer, and polyethylene terephthalate (PET)-copolymer.

18. Multicompartment bag according to any preceding claim, **characterized in that** the breakable welds are opened by manually applying pressure to at least one of the compartments separated by the breakable weld.

19. Multicompartment bag according to any preceding claim having one or more ports.

20. Multicompartment bag according to any preceding claim, **characterized in that** it is enclosed in an outer sealing envelope.

21. Multicompartment bag according to claim 20, **characterized in that** the outer sealing envelope has oxygen scavenging properties.

22. Multicompartment bag according to claim 19, **characterized in that** an oxygen scavenging composition is placed in the space between the multicompartment bag and the outer sealing envelope.

23. Multicompartment bag according to claim 22, **characterized in that** the oxygen scavenging composition contains fine iron powder covered with sea salt and a natural zeolith impregnated with a NaCl-solution.

24. Method for the preparation of a multicompartment bag according to any preceding claim comprising in a first step filling the stabilized Fe (III) preparation into one of the at least two compartments and sealing the compartment, and in a second step filling one or more aqueous diluents suitable for infusion separately into one or more compartments of said multicompartment bag.

25. Method according to claim 24, wherein the stabilized Fe(III) preparation is filled into one of the at least two compartments through an inlet port.

26. Method according to claim 17, wherein the stabilized Fe(III) preparation is filled into one of the at least two compartments without an inlet port.
